# EUROPEAN PATENT APPLICATION

(11) **EP 4 138 085 A1**
(43) Date of publication of application: **22.02.2023**
(21) Application number: 21191791.9
(22) Date of filing: 17.08.2021
(51) Int. Cl.: G16H 10/60, G16H 15/00

(54) **MANAGING A MEDICAL RECORD OF A SUBJECT**

(71) Applicant: Koninklijke Philips N.V., 5656 AG Eindhoven (NL)
(72) Inventor: BULUT, Murtaza, 5656 AE Eindhoven (NL); SARTOR, Francesco, 5656 AE Eindhoven (NL); VAN LIESHOUT, Ron Martinus Laurentius, 5656 AE Eindhoven (NL); MAESSEN, Ralph Theodorus Hubertus, 5656 AE Eindhoven (NL)
(74) Representative: Philips Intellectual Property & Standards

(57) **Abstract**

Methods and systems for managing a medical record of a subject. A method comprises: using (202) a first model trained using a machine learning process to determine whether to transform the medical record from a first form into a second form. The first model takes as input risk factors associated with storing the medical record in the first form and provides an output that can be used to determine whether the risk is decreased if the medical record is transformed into the second form compared to if the medical record is not transformed into the second form, and if it is determined that the medical record should be transformed into the second form, initiating (204) transformation of the medical record into the second form.

## Description

### FIELD OF THE INVENTION

Embodiments herein relate to medical records. Some embodiments relate to managing a medical record of a subject.

### BACKGROUND OF THE INVENTION

There are different tools used for patient care. Some of these tools relate to patient data storage and visualization, and interaction solutions (including paper printouts). They are used for sharing and discussing essential medical information, and for legal purposes.

A medical record can be stored and used in a variety of forms such as digital, paper, and/or different digital model representations. Dependent on the form of the medical record used by the clinician(s) treating subject (e.g. patient) there may be different outcomes for that subject. For example, if medical records are maintained in a purely digital manner, then this can leave patients vulnerable in the event of technical failure; if a clinician only has records stored in a digital text-based file, then this may result in poorer outcomes for the subject compared to if a relevant model were generated for the subject from the medical record; and if only paper formats are used, then this can be environmentally unfriendly and can lead to information loss if the paper records are inadequately maintained and/or updated. Paper records can further result in delays if the records have to be manually retrieved and searched.

### SUMMARY OF THE INVENTION

As described above, there are a variety of forms that medical records can be stored in, e.g. digital, paper-based, models and/or images of different types (2D/3D/4D etc). There are a variety of risks associated with only storing medical records in a subset of the different forms available e.g. due to technical failure, missing data etc.

One solution might be to produce all types of record in one go, for every subject, e.g. a digital copy, a print copy and/or a plurality of different models. However, this can have environmental implications, e.g. through the excessive use of paper print outs. Furthermore, storing all different formats requires greater physical and/or digital space.

There is also a general desire to go paperless, however despite the introduction of electronic medical records, analyses reveal that paper prints are still valuable and popular. There are thus advantages and disadvantages of both digital and paper prints.

Another consideration is that too many forms (or copies) of a medical record can lead to information overload for the clinician(s) treating the subject. This can result in decreased efficiency when searching for certain information, for example, increasing search times, increasing the risk of accessing and using outdated information and increasing the risk of missing important information. Thus, different forms of records (digital, paper etc) are valid and necessary depending on factors such as the treating clinician(s), different hospital and infrastructure settings, regulations, and working conditions.

As noted above, there are risks associated with maintaining medical records in a single form (e.g. only paper, only digital, etc), yet there are also disadvantages to producing medical records in all available forms as this can be less environmentally friendly, lead to confusion/information overload for clinical staff. Embodiments herein enable faster access to the right patient information. Embodiments herein propose to address these problems by determining the form(s) in which a medical record for a subject should be provided to and accessed by a clinician in order to reduce risks (which might be associated with accessing medical records in a single form) and thereby improve patient outcomes. Embodiments herein further improve resource usage in hospitals. This is a direct effect of having timely access to the right patient data, which enables more accurate diagnosis, and therefore more accurate treatment planning, directly resulting in improved utilization of hospital resources such as equipment, special location (such as labs, surgery rooms, bed allocation) and hospital staff

Embodiments herein propose machine learning (ML) based solutions for determining risks associated with not having certain data generated (e.g. as paper records) or of having certain interactive elements missing (e.g. 2D vs. 3D), and the benefits of having data generated and having various options to visualize and interact with the data (e.g. virtual reality environment vs. paper record). Some embodiments, as will be described in more detail below, use ML to determine when and what patient data records should be generated. This can have the benefit to of improving patient care by providing the correct information in the correct (or optimal) form at the correct time, thus leading to uninterrupted and more interactive patient care.

Thus, according to a first aspect, there is provided a method performed by a computing system for managing a medical record of a subject. The method comprises using a first model trained using a machine learning process to determine whether to transform the medical record from a first form into a second form, wherein the first model takes as input risk factors associated with storing the medical record in the first form and provides an output that can be used to determine whether the risk is decreased if the medical record is transformed into the second form compared to if the medical record is not transformed into the second form; and if it is determined that the medical record should be transformed into the second form, initiating the transformation of the medical record into the second form.

According to a second aspect there is a system for managing a medical record of a subject. The system comprises a memory comprising instruction data representing a set of instructions, and a processor configured to communicate with the memory and to execute the set of instructions. The set of instructions, when executed by the processor, cause the processor to: use a first model trained using a machine learning process to determine whether to transform the medical record from a first form into a second form, wherein the first model takes as input risk factors associated with storing the medical record in the first form and provides an output that can be used to determine whether the risk is decreased if the medical record is transformed into the second form compared to if the medical record is not transformed into the second form; and if it is determined that the medical record should be transformed into the second form, initiate transformation of the medical record into the second form.

According to a third aspect there is a computer program product comprising computer readable medium the computer readable medium having computer readable code embodied therein, the computer readable code being configured such that, on execution by a suitable computer or processor, the computer or processor is caused to perform the method of the first aspect.

In this manner, risks associated with having a medical record stored (only) in first form and not in a second form can be reduced. This leads to better management of the medical record, fewer risks to the patient care and improved patient outcomes.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 is an example apparatus according to some embodiments herein;
Fig. 2 is an example method according to some embodiments herein;
Fig. 3 is a flow chart illustrating example inputs and outputs of first and second models according to some embodiments herein;
Fig. 4 is an example flow chart according to some embodiments herein;
Fig. 5 is an example machine learning architecture according to some embodiments herein; and
Fig. 6 is an example machine learning architecture according to some embodiments herein.

### DETAILED DESCRIPTION OF EMBODIMENTS

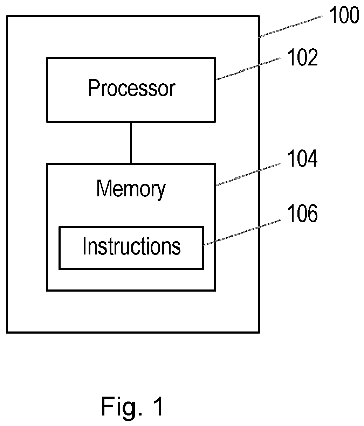

Fig. 1 shows an apparatus 100 for use in for managing a medical record of a subject, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method 200 as described below.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively, or in addition, one or more memories 104 may be external to (i.e. separate to or remote from) the apparatus 100. For example, one or more memories 104 may be part of another device. Memory 104 can be used to store any information or data received, calculated or determined by the processor 102 of the apparatus 100 or from any interfaces, memories or devices that are external to the apparatus 100. The processor 102 may be configured to control the memory 104 to store the information or data received calculate or determined by the processor.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

As will be described in more detail below, the apparatus 100 may form part of a system for managing a medical record. The set of instructions, when executed by the processor, may cause the processor to use a first model trained using a machine learning process to determine whether to transform the medical record from a first form into a second form, wherein the first model takes as input risk factors associated with storing the medical record in the first form and provides an output that can be used to determine whether the risk is decreased if the medical record is transformed into the second form compared to if the medical record is not transformed into the second form; and if it is determined that the medical record should be transformed into the second form, initiate transformation of the medical record into the second form.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

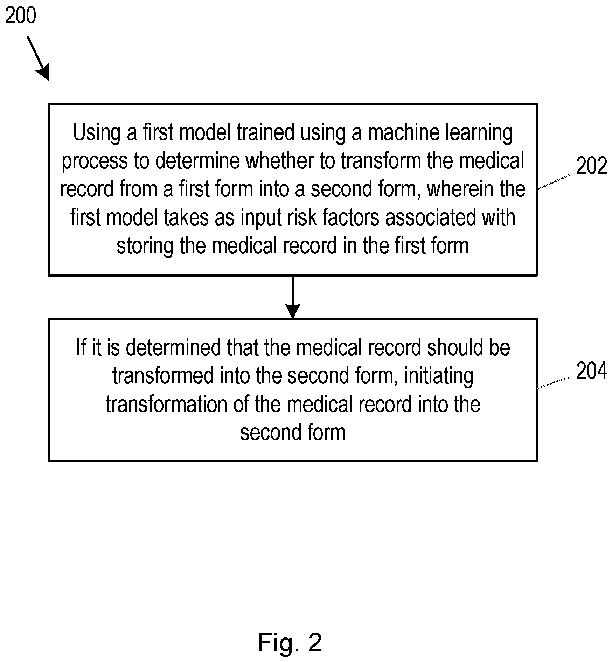

Turning to Fig. 2, there is a computer implemented method 200 for use in managing a medical record of a subject. Embodiments of the method 200 may be performed, for example by an apparatus such as the apparatus 100 described above.

Briefly, in a first step 202, the method 200 comprises: using a first model trained using a machine learning process to determine whether to transform the medical record from a first form into a second form. The first model takes as input risk factors associated with storing the medical record in the first form and provides an output that can be used to determine whether the risk is decreased if the medical record is transformed into the second form compared to if the medical record is not transformed into the second form. In step 204, if it is determined that the medical record should be transformed into the second form, the method 200 then comprises initiating transformation of the medical record into the second form.

Thus, the methods herein can be used to determine when to transform a medical record into another form (or format) so as to reduce risks associated with storing or accessing the medical record (only) in the first form; or not in the second form. This improves the access to the data e.g. in terms of access time.

As referred to herein, a subject (otherwise referred to as a patient) is generally a person or animal. The subject may be undergoing treatment at a medical facility, hospital, clinic or the like. As such the subject may be thought of as a patient of the medical facility, hospital, or clinic.

A medical record (e.g. medical data) may comprise any information held or generated (e.g. by monitors and other sensors) about the subject by the medical facility, e.g. for the purpose of providing care and/or treatment to the subject. The medical record may comprise information, for example, relating to health assessments carried out on the subject (observations and the like), details of symptoms of the subject, diagnoses made for the subject and/or treatments provided to the subject. The medical record may comprise signals, lab data, nursing notes, clinician notes, standard operating procedures and protocols, guidelines, workflow information, lifestyle data (e.g. data collected outside the healthcare context, e.g. in the wild) data collected from questionnaires, from sensors, and/or other people. The medical record may further comprise relevant population data. The medical record may be an electronic health record (EHR).

The medical record may be in a first form. In some examples, the form may be a format such as a digital format or a paper format. In other examples, the first form may be a medical (or anatomical) model or the output of a medical (or anatomical) model e.g. a medical model instantiated or configured based on medical data pertaining to the subject. Some example medical models include but are not limited to:
- Personalized models of blood flow, e.g. to plan valvular interventions such as valve repair or replacement for aorta or mitral valves.
- Electro-mechanical Heart models, geometric models of heart, digital twin models of the heart.
- Brain aneurism models.
- Tumor growth models.
- Tissue deformation models.
- Lung models, etc.
- Models for breast cancer: tumor diameter growth, cell invasiveness
- Osteoporosis: molecule-to-cell, cell-to-tissue coupling models
- Biomechanical models of hip joint
- Finite element model for knee joint kinematic behavior
- Finite element model for prostate deformation during biopsy

Further examples are provided in the paper by Neufeld et al. (2018) entitled "Functionalized Anatomical Models for Computational Life Sciences"; Front. Physiol., 16 November 2018.

In some embodiments the first form may be a digital twin. In other words, the medical record may be represented as a digital twin of the subject. The skilled person will be familiar with digital twins, but in brief, a digital twin is virtual representation of the subject, that may serve as a real-time digital counterpart of the subject. A digital twin has been defined as "a set of virtual information constructs that mimics the structure, context and behaviour of an individual or unique physical asset, that is dynamically updated with data from its physical twin throughout its life-cycle, and that ultimately informs decisions that realize value. See paper by Niederer, S.A., Sacks, M.S., Girolami, M., Willcox, K., "Scaling digital twins from the artisanal to the industrial", Nature Computational Science volume 1, pages 313-320 (2021).

Although the definition in Niederer et al. targets engineering systems, a broad interpretation covers the use of digital twins in a diverse array of other application areas such as healthcare and information systems. In such applications, the "asset" referred to in the definition may be interpreted to comprise processes and living entities, and the "lifecycle" to be the period over which the digital twin is needed to support decision-making. With this broad interpretation, the definition also covers cases in which digital twins are used over fixed time periods, such as for surgery or in critical decision points for physical systems.

In essence, a digital twin can be an in silico model that brings together the technology to map, monitor and control real-world entities by continually receiving and integrating data from the physical twin. It is noted however that the definition of digital twin is generally dependent on the application.

In some embodiments herein, a digital twin may be defined in a similar manner to Niederer et al., as a digital representation (in the virtual world) of a physical person (in the real world) that is dynamically updated during (a part of) the lifespan of its physical version via a two-way coupling by means of in-silico intelligence.

In broader terms, the term "Patient Digital Twin" can be as a framework of methods and technologies that once established will enable the doctor to look at the patient in a diagnosis-treatment episode as a single complex system. This framework can be descriptive (which includes all relevant data available of each individual patient), integrative (supporting longitudinal decision-making during the entire care-path where the patient is seen as a whole based on latest available data, updated dynamically before and during treatment execution), and the third element is predictive (which assists the doctor in using available knowledge to know in advance the expected outcomes of available treatment options before or during the therapy delivery, including the clinical outcomes, quality of life and costs).

In some embodiments, the first format may be a sequence of 2D images. For example 2D images may be converted into a 3D model (e.g. in some embodiments the first form may be a 2D image sequence and the second form may be a 3D model).

This may be used in imaging where 2D images are used to create 3D models, so that localization and navigation through different structures is easier. Examples can be taken from MR imaging, US imaging and others. One example is prostate cancer, where Magnetic resonance imaging (MRI) images are used to create 3D images, and also where Ultrasound (US) images are mapped to MR Images.

For ablation, having 3D models and fusion of different (real-time and stored) image modalities is helpful, for example, to prevent damage to surrounding structures by having more control over the ablation margins. For a patient, where the suggested ablation already is larger or closer to critical structures having such 3D models would be more essential than for another patient where the risk of damage is less.

In step 202 the method comprises using a first model trained using a machine learning process to determine whether to transform the medical record from a first form into a second form, wherein the first model takes as input risk factors associated with storing the medical record in the first form. The model provides an output that can be used to determine whether the risk is decreased if the medical record is transformed into the second form compared to if the medical record is not transformed into the second form.

The second form may be any of the forms described above with respect to the first form. For example, the second form may be a format such as a digital format or a paper format. In other examples, the second form may be a medical model or the output of a medical model e.g. a medical model instantiated or configured based on medical data pertaining to the subject. In some embodiments the second form may be a digital twin.

Further examples include but are not limited to the second form being:
- paper records of subject data, or clinician notes
- 3D, Augmented Reality (AR), Virtual Reality (VR), or Mixed Reality (MR) visualizations of subject data
- AI output resulting from processing subject data
- Digital model (digital twin) of the subject, or of a device, or of a process, or of a procedure , which is or will become part of the subject treatment

Generally, the second form will be different to the first form.

In some examples, the first form may be a digital form and the second form may be a paper form. In other words, the first model may determine whether a digital medical record should be printed to form a print-out of the medical record.

In some examples, the first form may be a digital form and the second form may be a medical or anatomical model or a digital twin of the subject. In other words, the first model may determine whether to create a digital twin or instantiate a medical or anatomical model from information in the medical record. As an example, in embodiments where the first form is a digital form and the second form is a medical model, step 206 may comprise obtaining a defined bio-physical model, and using the patient data in the digital medical record to personalize the bio-physical model.

Turning to the first model, the skilled person will be familiar with machine learning and models that can be trained using machine learning processes. The first model, otherwise referred to herein as a machine learning model, may comprise procedures for how to use data input to the model to e.g. make a prediction, or classification. Machine learning models are trained using machine learning processes on training data, and the resulting trained model represents what was learned during the training. The model represents e.g. rules, numbers, and any other data structures (probabilities, distributions, interactions or features) or architecture required to e.g. make predictions.

Examples of models that can be used herein include but are not limited to: decision tree algorithms, graph structures, neural networks, and support vector machines. Examples of machine learning processes used to train such models include, but are not limited to back propagation, gradient descent and logistic regression.

In some examples, the model is a Neural Network. In other examples, Bayesian methods may be used.

The models are trained using training data comprising training examples, each training example comprising an example set of input parameters and a ground truth (e.g. "correct") output for the example input parameters. The models herein may thus be trained in a supervised learning manner. The training dataset may be compiled from databases of medical records, and annotations provided e.g. by a human (e.g. a physician, medical expert or hospital administrator), as described below.

Models may be set up and trained using standard libraries and tools. Examples include the Scikit-learn libraries described in the paper: "Scikit-learn: Machine Learning in Python", Pedregosa et al., JMLR 12, pp. 2825-2830, 2011 and Google's Tensor Flow library described in the paper: "TensorFlow: Large-scale machine learning on heterogeneous systems" Abadi et al. 2015 (Software available from tensorflow.org).

In some examples, the first model outputs a determination of whether to transform the medical record from the first form into the second form. As an example, the determination may be a binary output (e.g. 1 or 0, corresponding to "yes" or "no").

The first model may be trained using training data comprising training examples, where each training example comprises i) an example medical record of an example subject; ii) example risk factors for the example subject; and iii) a corresponding ground truth determination of whether the example medical record should be transformed into the second form.

In another example the first model outputs: a measure (e.g. an indication) of risk associated with storing the medical record in the first form; and/or a measure (e.g. an indication) of benefit associated with transforming the medical record into the second form. For example, the first model may be a regression model that outputs a predicted percentage benefit to patient outcomes associated with transforming the medical record from the first form to the second form. As another example, the first model may be a classification model that grades the benefit according to predetermined levels of benefit, e.g. "high" benefit, "medium" benefit or "low" benefit, for example. The measure of risk/benefit may then be used to determine whether to transform the medical record (e.g. based on the risk preferences/tolerances of the medical facility).

In such examples, the first model may be trained using training data comprising training examples; wherein each training example comprises i) an example medical record of an example subject; ii) example risk factors for the example subject; and iii) a ground truth risk score associated with storing the medical record in the first form; and/or a ground truth benefit score indicating a benefit associated with transforming the medical record into the second form.

As another example, the first model may be trained to further take the first form as input and output an indication of one or more forms that the medical record should be transformed into. For example, if the medical record is in a first form "digital" then the first model may provide an output of "paper" indicating that the medical record should be converted into paper format.

It will be appreciated that the inputs and outputs described above are merely examples and that the first model may be trained to provide any suitable output that can be used to determine whether to transform the medical record from the first form into the second form.

The training data may comprise a large sample of annotated training examples. The training examples may be annotated (e.g. the ground truth may be provided by) by a human annotator, who, based on their understanding of the risk factors, provides a ground truth label for each training example. It will be understood that the labels may change e.g. between different medical facilities and/or based on the location of the medical facility. For example, the ground truth annotations may take into account the preferences, practices or procedures defined for the clinical facility in which the model is to be used and/or the regulatory requirements that the medical facility operates under (which may change depending on the location of the medical facility). Thus, models for different medical facilities may be trained to produce different outputs for a subject, dependent on the preferences/practice/regulatory requirements of the respective facilities.

In some embodiments, some of the training data may be labelled (e.g. annotated) in a semi-automated manner. For example, if there is a legal requirement to print if the subject is pregnant (or more generally matches some criteria) then all training examples relating to pregnant subjects (or subjects matching the criteria) may be automatically labelled for transformation into printed form. Furthermore, if previous clinician interaction with the data is stored, then features extracted from these interaction moments (e.g. time, duration, type) can be used for the annotations.

Such rule-based annotations may be used to label a portion of the data, whilst other training examples that cannot be annotated in an automated manner may be flagged for review and labelling by a human.

The skilled person will appreciate that large and varied training data sets are needed to reliably train models using machine learning processes. Thus, the training dataset should generally be compiled accordingly, with a good range of different example input values and different output classifications.

As noted above, in embodiments herein, the first model takes as input risk factors (e.g. risk parameters) associated with storing the medical record in the first form. The risk factors may comprise risks to the subject, e.g. associated with human factors arising from a doctor having information in a non-ideal form, regulatory risks indicating e.g. non-compliance if the medical record is held only in the first form and/or technological risks, e.g. associated with technical failure.

In some embodiments, the risk factors are indicative of whether there is a risk that care outcomes for the subject will be reduced if the medical record is provided to a clinician in the first form compared to if the medical record is provided to the clinician in the second form. For example, the risk factors may indicate:
i) that the subject has a complex medical condition. In such an example, the second form may provide context or additional information to aid a clinician to make a more informed choice.
ii) that the subject requires medical attention within a predetermined time interval, for example "urgently" or "immediately". In such an example, the second form may also condense, provide context or specify information to aid a clinician to make a more informed choice, more quickly. This can mean dimensionality reduction, complexity reduction, or simplification of patient information, or highlighting specific information that needs to be paid attention to (allergies, types of medications administered, previous procedures performed etc). For example, if the patient data is stored and accessed as a digital twin (first form), in this instance this can be transformed into list of items (second form) specific to the current patient condition, such as allergies, medications, and/or previous treatments.
iii) that the clinician (e.g. the clinician overseeing the care provided to the subject) has a preference to receive the medical record in the second form over the first form. In such scenarios, it may be beneficial to convert the medical record into the second form, for example to improve the clinician's understanding or workflow.

In other examples, the risk factors are indicative of whether: there is a risk of the medical record becoming inaccessible in the first form due to e.g. technical failure. In this sense, a technical failure may be any risk of the data getting lost, modified or otherwise corrupted. Technical failure may be due, for example, to a software failure, third-party attack, e.g. a ransomwear attack, environmental factors, e.g. flooding of a data warehouse. Where there is risk of technical failure, and the first form is a digital form, then the first model may be trained to indicate that the medical record should be transformed to a paper form (e.g. that it should be printed).

In some examples, the risk factors may be indicative of whether there is a data security risk associated with storing the medical record in the first form and/or the second form. These risks may include risks associated with the data getting lost, stolen, or modified.

In some examples, the risk factors may further indicate whether there is a regulatory risk associated with storing the medical record in the first form and/or not storing the medical record in the second form. For example, if there is a regulatory requirement in force for the medical facility that the subject is attending. For example, different countries have different legal requirements with respect to generating and storing health records in a paper format. For example, in the Intensive Care Unit (ICU) in Italy there is a requirement to generate printed records for 20/30 minutes of ECG before/after a person dies. In India, there is a requirement to generate paper records during child delivery.

As described above, in some embodiments, the first form is a digital record and the second form is a paper print out. In these embodiments, risk factors may be split into human risk factors and non-human risk factors. These may be described as follows.

Risk factors relating to non-human factors (associated with converting a medical record

### from a first (digital) form to a second (paper) form)

Non-human risk factors may comprise, for example, legal aspects, hardware and software components of the computational resources of the hospital, utility and/or structural properties.

The main advantages of having paper prints are risk (e.g. cost) and security. First, considering the risk:
There are certain situations where not having paper prints can be very risky. One case is when it is legally required to have prints. For these situations the decision of generating a print and what should be printed is straightforward and is based on pre-determined (legal) rules. The same considerations also apply to the hospital-specific local rules and regulation.

Computation resources, and software and the software environment enabling access to health data of the hospital are other factors that can be considered. There are many (technical - device or software related) problems that can limit or prevent proper operation and usage of complex electronic computational resources. Some of these are:
- Access to data only allowed to specific individuals
- External attacks that compromise security
- Connectivity problems that make patient data access slow
- Technical (hardware and software) problems that prevent access to patient data

Not having access to patient data when needed can be risky, as it can result in the patient receiving the wrong treatment or not receiving a treatment on time. Generally, it is desirable to generate paper prints before problems occur. This necessitates predicting their occurrence.

Some examples of how the first model may be trained to determine whether a medical record should be transformed from a first digital form into a second paper form are given below. To train the first model in the manner described below, training data may be labelled with ground truth "print" or "don't print" according to the principles below. In these examples, non-human factors are used to determine whether the medical record should be transformed from digital form to printed form (e.g. whether the digital record should be printed).

Risk Factor: Access to data. Access to data can be determined by knowing or estimating the people involved in the care of the subject. When some clinicians in the care team do not have direct access to the system to access the electronic health records paper prints should be generated. For example, this may happen, if there are clinicians involved from different hospitals or hospital units, etc.

Thus, one input to the first model may be an indication of whether all clinicians involved in care of the subject have access to the digital system. If they do then the first model may be trained to output that the medical record does not need to be transformed into the second (paper) form. If they do not, then the first model may be trained to output that the medical record should be transformed to the second paper form.

Risk Factor: External Attacks. External attacks can be predicted based e.g. on detecting unusual (software) activity. Thus, one input to the first model may be an indication of whether an external attack is predicted. If the indication is that an external attack is predicted, then the first model may be trained to output that paper records should be generated. Generally, in cases where a security breach is estimated in the electronic systems, they should be disabled, and paper records should be printed.

Risk Factor: Slow connectivity. Slow connectivity can be predicted based on number of patients, number of people accessing patient records, scheduled software updates, and extensive computations, such as digital twin simulation, planned to be performed. Thus, one input to the first model may be an indication of whether connectivity is predicted to be slow. In such cases, the first model may be trained to indicate that the medical record in the first (digital) form should be transformed to the second (paper) form. The first model may further take as input an indication of criticality of the subject's condition. In such a case, if slow connectivity is predicted, the first model may be trained to provide an indication that subjects in a critical condition, and/or non-critical subjects with a scheduled action such as medication intake, lab test, or surgery should have their medical records transformed from the first digital form into the second paper form.

Risk Factor: Technical Issues. Technical problems can be difficult to predict. But there is always a risk of them. In a modern hospital the risk is generally very small, but not zero. Because it is a non-zero risk, the first model may be trained to determine that medical records of the most critical subjects (e.g. 1% of critical subjects) should always be generated. Another example is to use data from past failures (e.g. including connectivity data, number of users and subjects, applications running, devices uses, etc.) to train the first model to probabilistically estimate the risk of technical failure. When the risk is higher than a set (e.g. predetermined) threshold, the digital records should be transformed to printed form. E.g. paper prints should be generated.

Risk factor: utility. Utility in this sense may refer to electricity. In case of a power failure, a power generator will be activated. The level of the generator capacity can be monitored to determine, when certain electronic systems will become unavailable, or available with a limited capacity or with a slow connection. The first model may be trained to take into account the unavailability of utility systems. For example, if there is high levels of electrical outage, then the first model may be trained to determine that the medical record should be transformed from the first digital form to the second paper form.

Risk Factor: Patient Care Location. In certain parts of a medical facility (e.g. hospital), such as the basement, wireless internet connection can be slow or unreliable. This may also apply to shielded rooms (such as MRI/CT) where wireless connection can also be unreliable. Knowing that access to patient data will be required from these places, the first model may be trained to determine that medical records should be transformed from the first digital form to the second paper form if the patient location is known to be in an area of the medical facility that experiences low connectivity levels.

Risk Factor: Security of patient data. In view of privacy regulations, data breach can have serious consequences. To prevent these, the first model may be trained to restrict printing of paper records based on patient location, to parts of a medical facility that are only accessible to clinicians, and/or to times outside visiting hours. The level of security can also be balanced by the risk/benefit principle. For example, when the utility situation is critical the level of restriction related to privacy and access of the data can be reduced to allow for a higher certainty that the paper will be printed.

Risk Factor: Infection Control. Risk of infection spreading due to the use of common electronic devices can be another factor input to the first model. For example, where there is a prevalence of an infectious disease, such as Covid-19, the first model may be trained to determine that medical records in the first digital form should be transformed to the second printed form to limit the number of clinicians touching same electronic equipment. For example, personal paper prints may be recommended. For example, the first model may be trained so as to determine that a medical record for the attending clinician who is responsible for many patients (and/or at different hospital units) is to be transformed into paper form, e.g. delivered via paper prints during his bedside visits, while for the ICU nurse who is working in the confined ICU space, the patient information can be communicated electronically via a personal computer, without paper records. To prevent infection spreading via paper prints, separate prints for different users may be generated. Similar considerations are also applicable for the use of laboratories where various tests are conducted. The access and usage of the devices in the laboratory space can be limited only to the people continuously working in the laboratory, and for other clinicians that need to use the laboratory in an on-and-off manner, the information can be prepared in a paper format. In that way, the risk of potential infections (transmitted via using same electronic devices in the lab) can be reduced. Undetected or uncontrolled infection in a medical facility can have serious and even deadly consequences. By making use of paper records, such risks may be reduced.

### Risk Factors related to Human Factors (associated with converting a medical record from a first (digital) form to a second (paper) form)

Alternatively, or additionally, the first model may be trained to take as input risk factors related to human factors. It can be advantageous to patient outcomes to provide paper print outs to clinicians in various circumstances. For example, printouts may reduce cognitive load in comparison to finding and viewing information digitally (due to ease of finding information, and familiarity). Print outs may improve ease of annotation, discussion, and sharing with team members. Print outs may give more time and opportunity for face-to-face interaction with the subject (patient). A clinician may pay more attention to medical records presented in printed form (due to defined paper size, and limited amount of information). Medical records in printed format may potentially be able to provide more tailored and relevant information (to limit the impact of data breach if paper is lost), hence making it more difficult for clinicians to miss or ignore essential information.

Thus, the first model may be trained to determine whether there are risks associated with not transforming the medical record from the first (digital) form to the (second) paper form, due to human factors that may improve care if the medical record is provided to a clinician in paper form as opposed to digital form.

Risk factors related to human factors may be collected and used in an automated manner.

For example, clinicians may indicate and store their preferences (of wanting or not wanting paper records). The stored preferences may then be provided to the first model as input for use in determining whether the medical record should be transformed from the first form to the second form.

Further risk factors/inputs to the first model may be obtained from e.g. digital calendars and/or other scheduling tools. For example, scheduled information of planned team meetings and discussions are objective and easily assessable sources of information which can be used. For example, the first model may be trained to determine that the medical record should be transformed to the second paper form ahead of meetings and/or for patient cases/conditions where discussion is expected. The unknowns in the patient condition (in comparison to the similar patients or conditions treated by the clinicians) can be used to automatically estimate the amount of discussions and annotations that may be needed. For instance, complex cases may require more annotations and therefore paper records of these can be preferred.

Studies indicate that some clinicians prefer paper prints to have more time for personal interactions with the patient which leads to improved patient care. This is also true for patients for some of which it may be more comforting (and maybe healing) to be able to communicate longer with the clinicians. Clinician preferences have been discussed above. Patient interaction preferences can be also stored and provided as input to the first model. Patient interaction preferences can be determined in various ways, for example, nurses can evaluate patients and store their interaction preferences in the system. It can be assumed that certain age groups (such as elderly) may require more interactions, and explanations. In certain instances, when the information to be communicated is difficult to understand, or emotionally sensitive, or a lot, the time needed for interaction can be larger, and in such instances the model may be trained to determine that medical records in digital form should be transformed to paper form.

Chronically ill subjects and/or subjects with a long history with a given caregiver may be more involved and aware of their medical records. In addition, they have a better familiarity with the caregiver and therefore may perceive of the need for paper to facilitate personal interaction.

Some research studies indicate that due to the well-defined and limited size of the paper, and the limited amount of information that can be printed, clinicians are more careful and attentive in examining the content, and the chances of them overlooking or ignoring (written) information is less. In other words, their attention is greater (i.e. seeing all information) and sustained for longer time (i.e. less distraction in comparison to online visualization). In view of these, to ensure that critical patient information is not ignored, the first model may be trained to determine that digital medical records should be transformed to printed form for critical patients. Especially in the periods where the clinician attentions is estimated to be low, for example due to time of day (especially during night shifts), long working hours, many patients to attend, shift change, etc. the first model may be trained to determine that digital medical records should be transformed to printed form paper prints. Moreover, the amount of information printed on the paper can be distilled to the essentials for the given time frame. This will increase the uptake of the information.

Studies also indicate that finding and reviewing information online can be cognitively more demanding that reading the same information from the paper. The periods of physical and mental tiredness of the clinicians can be estimated based on their workload, and the allowed cognitive load can be computed as a function of the mental and physical tiredness.

Thus, the method 200 may further comprise determining an estimation of cognitive load of a clinician and when the cognitive load is above a threshold (which may be personalised to the individual clinician) the first model may be trained to determine that digital medical records should be transformed to printed form paper prints.

Thus, based on different risk factors, a machine learning model may be trained to predict whether a medical record should be transformed from a first form into a second form.

Turning now to step 204 in Fig, 2, the method 200 then comprises: if it is determined that the medical record should be transformed into the second form, initiating transformation of the medical record into the second form. Step 204 may comprise transforming the medical record; or instructing another computing node or other computing equipment (e.g. such as a printer or display) to transform the medical record.

In this sense, transforming may involve converting the medical record into the second form. For example, if the first form is an electronic form and the second form is a paper print out, the step of initiating 204 transformation of the medical record into the second form may comprise sending an instruction to a printer to cause the printer to print the medical record.

As another example, if the second form is an anatomical model, the step of initiating 204 transformation of the medical record into the second form may comprise instantiating the anatomical model using parameter values obtained from the medical record.

As another example, if the second form is a digital twin, then the step of initiating 204 transformation of the medical record into the second form may comprise creating (or setting up) the digital twin using parameter values obtained from the medical record. Once set up, one or more simulations may be run on the digital twin.

The entire medical record does not necessarily need to be transformed into the second form each time. For example, the method 200 may further comprise determining (or selecting) which information in the medical record is to be transformed into the second form. In such examples the step of transforming the medical record into the second form thus comprises transforming the determined (or selected) information into the second form.

Machine learning may be used to determine which information should be included in the transformed medical record, for example, the step of determining which information in the medical record is to be transformed into the second form may be performed using a second machine learning model that takes as input the medical record, and outputs a determination of which information in the medical record is to be transformed into the second form.

The second model may be any of the types of model that were described above with respect to the first model. Furthermore, the second model may be trained according to any of the methods/processes described above.

For example, training data for the second model may comprise the following:
Input data: digital patient health records
Output data: content printed on the paper by human users
Considering the vast number of print outs generated in clinical settings (hospitals etc), training data is readily available, once the printed patient data is logged. In other words, the second model may be trained using the content of printouts generated by human users to determine which content is to be printed.

In other examples, in addition to patient data, data concerning non-human and human factors, explained above, as well as the intended audience (e.g. who the print out is for) can be also used as input data while training the second model.

In other examples, rule-based algorithms (such as look up tables) can be used, together with the machine-learning based training, that based on non-human and human factors determine the type and amount of data needed to be printed. This additional implementation is beneficial, because it gives a direct control to the clinicians to set rules for document content.

As another example, resources on report generation, information extraction and summarisation that employ artificial intelligence techniques such as natural language processing may be used to determine the content to be printed. Examples include but are not limited to those described in the following papers: Rimma Pivovarov & Noemie Elhadad (2015) entitled: "Pivovarov R, Elhadad N J Am Med Inform Assoc 2015;22:938-947"; Am Med Inform Assoc 2015;22:938-947; McInerney et al. (2020) "Query-Focused EHR Summarization to Aid Imaging Diagnosis", Proceedings of Machine Learning Research 1-26, 2020; Li et al. (2021) entitled "Neural Natural Language Processing for Unstructured Data in Electronic Health Records: a Review" (see e.g. section 6.2); and Rajkomar et al. "Scalable and accurate deep learning with electronic health records", npj Digital Medicine (2018) 1:18.

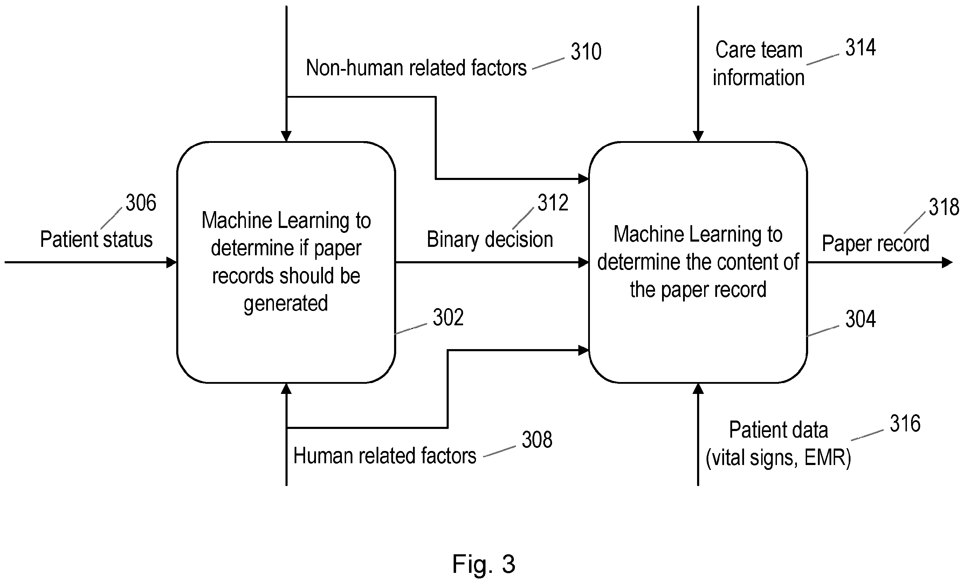

Turning now to Fig. 3, which shows an example embodiment whereby the first form is a digital record and the second form is a paper print out. In this example, the method 200 is used to determine whether a medical record in digital form should be printed (e.g. transformed into a paper record) and what should be included in the printout. As shown in Fig. 3, a first model 302 trained using a machine learning process is used to perform step 202 and determine whether to transform the medical record from the first form (digital) into the second form (paper print out). A second model 304 trained using a second machine learning process is then used to determining which information in the medical record is to be printed (e.g. transformed into the second form).

The first model 302 may be described as a binary decision unit that decides if a paper print is needed, and the second model 304 may be described as a content selection unit that determines the content of the paper prints. As noted above, in the example shown in Fig. 3, both units are based on machine learning models, although it will be appreciated that this is an example and that other processes could equally be used e.g. to determine the content of the paper print out.

In the example shown in Fig. 3, the first model 302 takes as input one or more fields from the medical record 306 and risk factors. In this example the risk factors may be related to human factors 308 and/or non-human factors 310 as described in detail above with respect to step 202.

The first model 302 is used to perform step 202 of the method 200 above and determine whether to transform the medical record from the first (digital) form into the second (paper) form.

Once the decision to print is made, the content needs to be determined. In the example illustrated in Fig. 3, a second model 304 trained using a second machine learning process is used to determine the content to be printed.

In Fig 3, a second model 304 trained using a second machine learning process is used to determine which information in the medical record is to be converted into the second form (e.g. which information is to be printed). Generally, the amount of information and the type of information can be objectively determined based on the number of complications, side-effects, and actions that a patient should (legally) be informed off.

In this example, training data comprises the following:
Input data: digital patient health records (care team information 314; patient data from medical records 316)
Output data: content printed on the paper by human users
Model 304 may be trained using the content of printouts generated by human users (and e.g. recorded in logs) to determine which content is to be printed.

The output of the example in Fig. 3 is that the information determined by the second model 304 is transformed into paper format (e.g. printed). It will be appreciated that Fig. 3 is an example only and that the principles therein may be generalised to other examples where the first and second forms are different to those illustrated in Fig. 3.

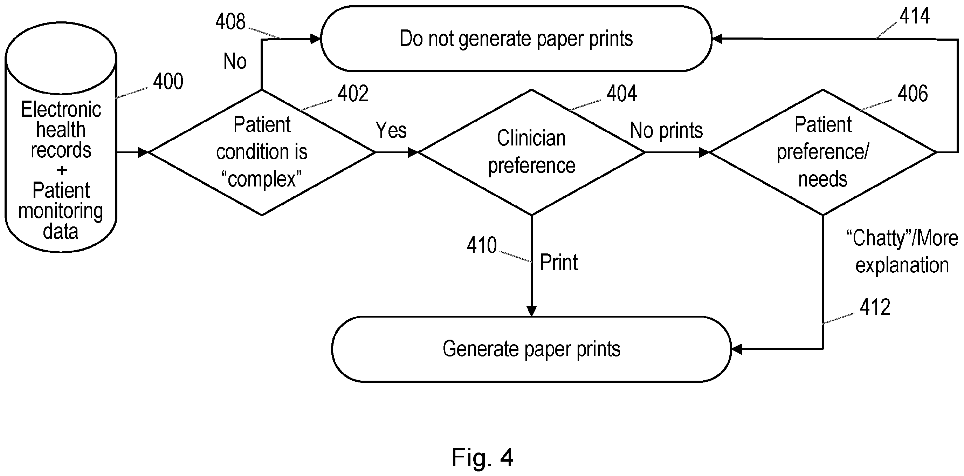

Turning now to Figs. 4-6 which illustrate three different example implementations of step 202 of the method 200 (e.g. the step of using a first model trained using a machine learning process to determine whether to transform the medical record from a first form into a second form wherein the first model takes as input risk factors associated with storing the medical record in the first form).

Three methods of determining whether to transform the medical record from a first (digital) form into a second (paper) form can be considered in a broad sense:
i) The first model may comprise a rule-based, decision tree like model. For example, with if-else rules that determine the path to be taken by comparing a measured or calculated values against pre-determined thresholds. The thresholds may be dynamic and can be adapted based on set frequency, for instance in a weekly manner. The thresholds may be calculated from past data. Threshold update times can be also dynamic and learned from data (e.g. the first model may be a decision tree).
ii) The first model may be a machine learning model such as a neural network model, where the weights for different factors are learned from past data, as described in detail above. The calculated or measured risk factors are used as inputs to the first model, and a binary output of print or no-print decision is made that is then used to determine whether to transform the medical record from the first (digital) form into the second (print) form.
iii) A hybrid method combining output of the first model with rule-based methods may also be also considered.

An example of a hybrid-based method implementation is shown in Fig. 4. In this example, there are three decision boxes evaluated to reach to a decision. Data on the subject is retrieved from the subject's medical record as stored in a database 400. First, subject-condition based evaluation is performed in step 402. If subject condition is classified as not complex, paper prints are not generated as illustrated in step 408. If the subject's condition is complex, then clinician preferences are checked in 404, and if clinician prefers paper prints these are generated in 410. In case clinician does not have preference for prints, the patient preferences and needs are considered in step 406. If the patient needs or requires more time and interaction paper prints are generated in 412, and otherwise not 414. In an alternative embodiment, for the case when clinician preference is for printing, instead of immediately generating the prints, a manual confirmation by the clinician can be required/obtained.

In the example shown in Fig. 4, one or more of the boxes 402, 404 and/or 406 may be implemented using a first model trained using a machine learning process (e.g. a first machine learning model). For example, the first decision module evaluating patient condition 402 may be a machine learning classifier that uses patient data from electronic health records and patient monitors to generate an output indicating the complexity of the patient state. If the existing data is sufficient to evaluate patient condition and the required treatment actions with high likelihood (i.e. given the data it is clear how the care should be provided) then complexity is low. On the contrary, if there are many unknowns, for example if the likelihood of clinical decision support systems is low, then the complexity is high. Examples of different implementations are the following.
- Patient similarity algorithms based evaluation: One way to implement such a system is based on the patient-similarity methods. If patient state is similar to past patients, the actions needed to be taken are assumed known, hence complexity is low.
- Change in vital sign signals: If the vitals are stable, the complexity is low
- Response to medication or treatment: If the response (i.e. change in vitals) is as expected, the complexity is low. Also, if the change of medical subscription (medication dose & type) is stable, the complexity is low.
- Medical history of the patient, consistent of the source of the patient and the underlying comorbidities. For example, if the patient originated from the emergency department and/or the patient has a lot of comorbidities and chronic illnesses.

As an example, the second decision box of clinician preferences 404 in Fig, 4 may be driven by stored clinician preferences. It may be implemented by asking clinicians to indicate their preferences of having printouts and storing the answers. This information may be stored as a part of the clinician's profile. In another embodiment, the clinician is asked in real time to print.

Alternatively, the second box 404 may be implemented using a machine learning model that predicts clinical preference based on past behaviour (e.g. previous printing decisions made by the clinician).

As an example, the third decision box 406 may be driven by patient needs and nature (i.e. amount and complexity) of information to be communicated. The patient needs may be stored in a profile for the subject. If patient is classified (e.g. by a clinician) as preferring/requiring more social interaction and the amount of information to be communicated requires more than 2 minute, paper prints may be generated.

The skilled person will appreciate that the decision process in Fig. 4 is merely an example and that other combinations of modules with different decision outcomes may equally be used, for example dependent on the preferences/risk tolerances of the medical centre in which the system is to be used. Furthermore, different modules may be implemented using machine learning and/or decision logic to those illustrated in Fig. 4. It will further be appreciated that Fig. 4 is an example only and that the principles therein may be generalised to other examples where the first and second forms are different to those illustrated in Fig. 4.

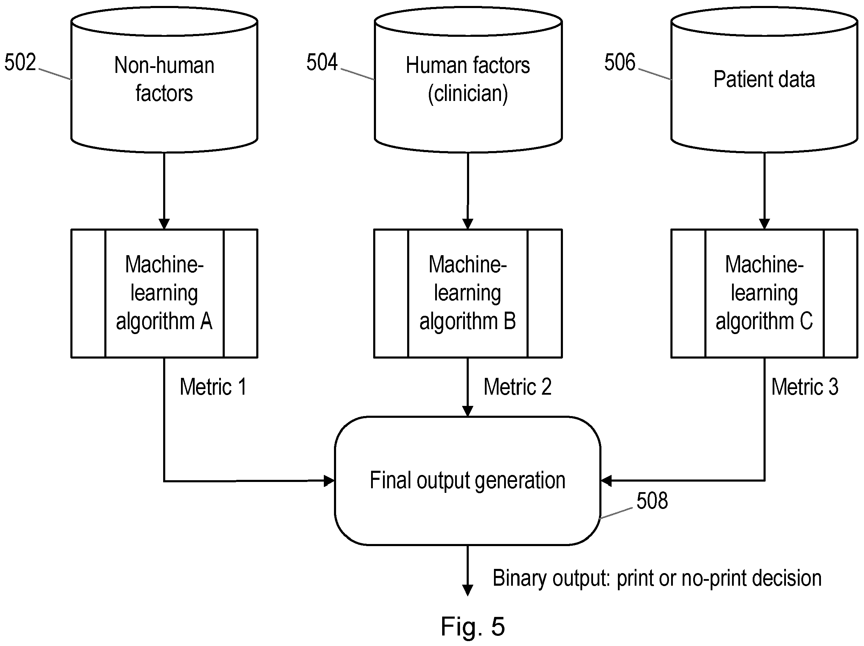

Turning to Fig 5, Fig. 5 shows another example manner in which to perform step 202 of the method 200. In this example, three models trained using machine learning processes are used to determine whether to transform a medical record from a first digital form into a second paper form. In Fig. 5, risk factors comprising non-human factors 502 and human factors 504 along with patient data from the medical record 506 are used to determine whether to transform the medical record from the first (digital) form into the second (paper) form. In this embodiment, three models are used, which are labelled A, B and C. For completeness, it is noted that in this embodiment, the first model may refer to any one or any combination of the models A, B and C.

In this example, model A takes as input non-human factors 502 and outputs a first metric; model B takes as input Human factors 504 and outputs a second metric and model C takes as input patient data 506 and out puts a third metric. The first, second and third metrics are then combined into a binary decision of whether the medical record should be transformed into print form.

With respect to non-human factors, as described above, the goal with these factors is to prevent health data from being inaccessible, or difficult to access. Therefore, in this example, model A may be trained using a machine learning process to predict a likelihood of data becoming electronically (e.g. digitally) inaccessible. The training input-output data pairs (e.g. training data comprising example inputs and corresponding ground truth outputs) may be constructed as follows:
- Input data: Objective measurements calculated or measured for the non-human factors discussed above, such as network speed and congestion, number of users, location of access, etc.
- Output data: Records of data access, indicating the records of data access requests by clinicians. These can be obtained from log files, indicating the time of login, time of first data display, time of specific data request, and time of the requested data display. In this example, the log data may be used to compute a first metric related to delays associated with accessing the requested information. As an example, the first metric may be e.g. an average time corresponding to the time delay of accessing requested information. The skilled person will appreciate that this is merely an example however and that other metrics could also be used for the first metric, such as, for example rate or number of data access fails.
- The machine-learning algorithm may then be trained to predict the first metric from the input data. Alternatively, the first metric can be further processed, and converted into a binary parameter by applying a mathematical function. For example, if the first metric is the average access time, this can be compared against a set time threshold (e.g. 30 sec), and all values larger than the threshold can be marked as 1, and the rest as 0. The machine-learning algorithm can be then be trained to predict the binary parameter from the input parameters, based on the labelled training examples.

Note that the input and output data described above can generally be collected and processed unobtrusively. By providing definitions of what input and outputs are needed, data collection can then be performed. After collecting sufficient data (which may be, for example, 2 months or more of data) from the hospital operations, the model can be trained.

With respect to model B, model B may be trained to determine/predict a second metric related to human factors. For example, the second metric may be a measure of the time it takes for a clinician to find the information they are looking for. Differently than non-human factors, here in addition to having access to health data, there is a second need, and that is to find specific information.

Time to access health data can be determined as described above, by using time from login to information request (e.g. login request + click patient name) and access (e.g. display of patient data). This can be also extended by considering the time of "need to access data". Data need start time can be determined from the triggers and alarm generated by patient monitors. For example, at the time an alarm is generated, "the need for data" starts. Time of patient data access can then be calculated as the difference between time patient data is displayed and data need start time.

Time to find the needed information (once the patient data is accessed) can be computed, for example, in an automated manner based on the following assumptions related to the user (e.g. clinician) behaviour.
- Assume that once the patient data is available, the clinician will start scrolling through different data screens looking for some specific information. This information is in clinicians' mind, and is unknown to the system. However, it can be assumed that once the searched information is found, the behaviour of the clinician will change. For instance, they will stop with scrolling, to pause and analyse the data. As such, a (long) pause can be taken as an indication that the searched information is found.
- By having records of the pause times and pause durations, the information access time can be estimated.
- In case it is known which information is being searched for, for example if there is search term entered by the clinician, or if the patient data access is linked to a particular type of alarm or defined patient condition, then the time of information access can be more accurately computed by recording the delay between search initiation, and needed information being displayed on the screen for a least a set minimum period of time.
- The second metric (information access time) can be directly used and model B can be trained to predict it using machine learning processes, as described above. As an alternative example, the time metric can be converted to a binary parameter, and model B can be trained to predict the binary value.

The input risk factors for model B comprise human related risk-factors. The desire is to have this data automatically and unobtrusively measured to minimize interruption and inconvenience to the clinicians. The following clinician related information can be used as inputs to model B.
- Working time related measures to determine the busyness at work, estimated tiredness, and drop in cognition:
- Time working, Shift start and end times
- Number of patients treated
- Number of rooms visited; number of steps taken
- Calendar information to estimate number of interactions with other clinicians
- Measurement of posture, gait, eye tracking, speech to estimate mental and physical tiredness level
- Measurement of computer usage, such as number of clicks, and typing speed to estimate tiredness.

Using the clinician data (from personal tracking devices, localization devices in hospital rooms, device usage, calendar information, hospital records, etc.) as input, and the time to information access as output, a model B can be trained using machine learning processes.

Model C is trained to take the medical record (e.g. data therefrom) as input and predict a third metric related to complexity or urgency of care required for the subject.

In step 508, the first second and third metrics are combined to form a binary output. The metrics may be combined in different manners depending on the intention of the medical facility using the model. For example, the metrics may be normalised and/or combined into a single final metric using a weighted combination. This final metric may be converted into a binary indication of whether the medical record should be transformed from a digital form to print form, using a threshold.

To summarise the embodiment of Fig. 5, model A is trained to predict a first metric related to potential delay in accessing the patient health data via digitally means, such as electronic health records; model B is trained to predict the potential delay in finding the required information in the patient health data; and model C is trained to predicts a third metric related to complexity/urgency of the subject's care, based on the medical record (e.g. patient data). Step 202 of the method 200 then involves using the three metrics as an input to a pre-determined mathematical function (e.g. set rules) to generate final decision in step 508.

It will be appreciated that Fig. 5 is an example only and that the principles therein may be generalised to other examples where the first and second forms are different to those illustrated in Fig. 5.

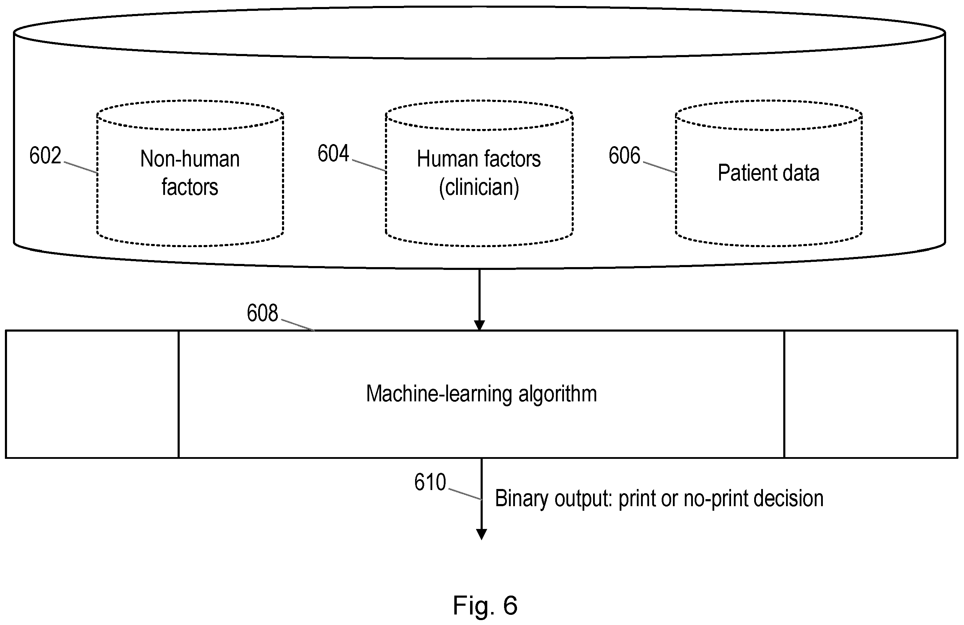

Turning to Fig. 6, in another implementation, in step 202, a single model 608, which may be referred to as the first model according to the terminology above, may be trained using a machine learning process to take a combination of risk parameters described above (e.g. a mixture of non-human factors 602, human factors 604 and/or patient data from the medical record 606) to directly generate a binary output (print/don't print) for use in step 202 for determining whether to transform the medical record from the first (e.g. digital) form to the second (e.g. print) form. In this example, the non-human factors and human factors may be gathered in the manner described above with respect to Fig. 5 and the detail therein will be understood to apply equally to the embodiment illustrated in Fig. 6.

It will be appreciated that Fig. 6 is an example only and that the principles therein may be generalised to other examples where the first and second forms are different to those illustrated in Fig. 6.

Turning now to other embodiments, in some embodiments of the method 200 where the method is for use in determining whether to transform a medical record from a first form comprising a digital form to a second form comprising a paper form, (e.g. for determining whether to print a digital record) the determination in step 202 may further be based on one or more of the following factors:

### Monitor size dependent decision

Other factors are physical devices used to display the patient data. In case there is a mismatch between the characteristics of these and the characteristics of the data that need to be communicated to the clinician, paper prints can be generated.

One example relates to the size/resolution of the monitors. When they are of insufficient size, the clinicians may have difficulties in viewing the data in the most efficient and productive manner: For example, if they need to toggle between different screens, and scroll a lot. In such cases, the paper prints can be very beneficial.

### Display monitor interactivity dependent decision

The user-interface characteristics can be also taken into account. In case it is estimated that the clinicians would need to make annotations on the patient data (e.g. when patient status is complex), and the equipment in the room where clinician is estimated to visualize the data does not support such interactivity (e.g. not a touchscreen monitor), then paper prints can be generated. Having paper prints will remove the inconveniences due to user interface because paper prints can be easily annotated.

### Legal status based decision

For traceability and legal reasons, a paper version of the medical record is required under certain conditions. The non-human factors of the algorithm & patient data are needed to decide if there is a need to print medical information for legal reasons. However, privacy considerations have to be taken into account and the amount of data that is printed should be limited to satisfy the underlying legal reasons for the paper print but it should not extend beyond this legal data limit/requirements in order to reduce the impact of a potential data breach.

### Alarm management

A use case could be that the nurse would need to go through the alarms, and as it usually the case many of them can be artefacts (i.e. false alarms). The system could already discard artefacts and only print information of the events which are likely to be true events. The print could be already formatted as a specific consultant would like to see it, Cardiologist, vs Neurologist etc. In other words, generated alarms can be post-processed to reduce number of false alarms, and for the remaining alarms paper prints can be generated. The content of the paper print will include patient data relevant to the alarm, and potentially for the mitigation of the alarm.

As a different implementation, instead of automatically filtering and discarding the potentially false alarms, alarm tags indicating the chance of them being false alarm or not can be generated. The end user can them decide if an alarm is a false alarm, and correspondingly for printing.

### Workflow related decision making

Workflow related elements can have an impact on the value and risk of paper. Several workflow elements can increase the risk of paper, for example if the paper is printed close to shift change the chance that the paper is not handled correctly is large and can even lead to loss of patient data.

During visitor hours the chance of a data breach is also higher and therefore the calculation between benefits/risks is also changed.

If the amount of measurement/data related to the patient has changed a lot in the given timeframe the benefits of providing paper records is also increased.

These factors can be incorporated into the printing or not printing decision.

### Making annotated paper print scans a component of medical records

As noted above, in many cases having paper prints can be advantageous. To facilitate traceability and keeping records, scans of the paper prints after they have been used can be made a part of the medical records.

There can be a procedure around this, such as before discarding the paper records, they are scanned and stored digitally. If the modification (including annotations) on the printed record are detected, the printed record can be digitized and stored, for example. This process can be easily performed by machines, because detecting annotations or modification to the original oriented document is possible. For example,
- The document can be compared with the original version
- The hand written text and annotations can be automatically detected and recognized.

### Making digital records in shape and format of pa^{p}er prints

One of the advantageous properties of paper prints, as shown in research studies, are their bounded and well defined format and dimensions, which can make it easier to search and locate information. Hence, similar to paper, information can be stored digitally but with well defined format, and bounded dimensions.

In some cases, the proposed invention can be used to generate paper prints not physically, but digitally. This means that, a decision to "print" (in this case this means to store) can be generated, and then "print content" (in this case this means the content to be stored) can be generated.

In some examples, the format (e.g. dimensions, margins, color, etc.) can be identical to the physical paper. The content can be selected so that it will fit in a readable manner to the paper. In other words, consider this as a digital version of a physical paper.

Having such a means to store and share information can be advantageous because the information will be available digitally while having the familiar and comfortable format of papers.

In other examples, the digital version can still have well defined dimensions and boundaries similar to physical objects, but these dimensional can be different that physical papers.

Turning now to other embodiments, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method performed by a computing system for managing a medical record of a subject, the method comprising:
using (202) a first model trained using a machine learning process to determine whether to transform the medical record from a first form into a second form, wherein the first model takes as input risk factors associated with storing the medical record in the first form and provides an output that can be used to determine whether the risk is decreased if the medical record is transformed into the second form compared to if the medical record is not transformed into the second form; and
if it is determined that the medical record should be transformed into the second form, initiating (204) transformation of the medical record into the second form.

2. A method as in claim 1 wherein the first model outputs a determination of whether to transform the medical record from the first form into the second form.

3. A method as in claim 2 wherein the first model was trained using training data comprising training examples; and
wherein each training example comprises i) an example medical record of an example subject; ii) example risk factors for the example subject; and iii) a corresponding ground truth determination of whether the example medical record should be transformed into the second form.

4. A method as in claim 1 wherein the first model outputs:
a measure of risk associated with storing the medical record in the first form; and/or
a measure of benefit associated with transforming the medical record into the second form.

5. A method as in claim 4 wherein the first model was trained using training data comprising training examples; and
wherein each training example comprises i) an example medical record of an example subject; ii) example risk factors for the example subject; and iii) a ground truth risk score associated with storing the medical record in the first form; and/or a ground truth benefit score indicating a benefit associated with transforming the medical record into the second form.

6. A method as in any one of the preceding claims wherein the risk factors are indicative of whether there is a risk that care outcomes for the subject will be reduced if the medical record is provided to a clinician in the first form compared to if the medical record is provided to the clinician in the second form.

7. A method as in claim 6 wherein the risk factors indicate that the subject has a complex medical condition, that the subject requires medical attention within a predetermined time interval, or that the clinician has a preference to receive the medical record in the second form over the first form.

8. A method as in any one of the preceding claims wherein the risk factors are indicative of whether:
there is a risk of the medical record becoming inaccessible in the first form due to a technical failure;
there is a data security risk associated with storing the medical record in the first form and/or the second form; and/or
there is a regulatory risk associated with storing the medical record in the first form and/or not storing the medical record in the second form.

9. A method as in any one of the preceding claims wherein the second form is a digital twin of the patient.

10. A method as in any one of the preceding claims wherein the first form is an electronic form and the second form is a paper print out; and
wherein the step of initiating transformation of the medical record into the second form comprises:
sending an instruction to a printer to cause the printer to print the medical record.

11. A method as in any one of the preceding claims wherein the second form is an anatomical model and wherein the step of: initiating (204) transformation of the medical record into the second form comprises instantiating the anatomical model using parameter values obtained from the medical record.

12. A method as in any one of the preceding claims further comprising:
determining which information in the medical record is to be transformed into the second form; and
wherein the step of initiating (204) transformation of the medical record into the second form comprises: transforming the determined information into the second form.

13. A method as in claim 12 wherein the step of determining which information in the medical record is to be transformed into the second form is performed using a second machine learning model that takes as input the medical record, and outputs a determination of which information in the medical record is to be transformed into the second form.

14. A system (100) for managing a medical record of a subject the system comprising:
a memory (104) comprising instruction data representing a set of instructions (106); and
a processor (102) configured to communicate with the memory and to execute the set of instructions, wherein the set of instructions, when executed by the processor, cause the processor to:
use a first model trained using a machine learning process to determine whether to transform the medical record from a first form into a second form, wherein the first model takes as input risk factors associated with storing the medical record in the first form and provides an output that can be used to determine whether the risk is decreased if the medical record is transformed into the second form compared to if the medical record is not transformed into the second form; and
if it is determined that the medical record should be transformed into the second form, initiate transformation of the medical record into the second form.

15. A computer program product comprising computer readable medium the computer readable medium having computer readable code embodied therein, the computer readable code being configured such that, on execution by a suitable computer or processor, the computer or processor is caused to perform the method as claimed in any one of claims 1 to 13.
